# EUROPEAN PATENT SPECIFICATION

(11) **EP 3 340 891 B1**
(45) Date of publication and mention of the grant of the patent: **14.10.2020**
(21) Application number: 16754154.9
(22) Date of filing: 17.08.2016
(51) Int. Cl.: A61B 17/02, A61B 17/88

(54) **APPARATUS FOR INTERNAL HIP DISTRACTION**
VORRICHTUNG FÜR EINE INTERNE HÜFTDISTRAKTION
APPAREIL POUR LA DISTRACTION INTERNE DE LA HANCHE

(30) Priority: 28.08.2015 US 201514838662; 28.08.2015 US 201514838723
(43) Date of publication of application: 04.07.2018
(73) Proprietor: Arthrex, Inc., Naples, FL 34108-1945 (US)
(72) Inventor: KELLY, Bryan, Riverside, Connecticut 06878 (US); SCHMIEDING, Reinhold, Naples, Florida 33963 (US); ALBERTORIO, Ricardo, Naples, Florida 34110 (US); HELENBOLT, Kenneth, Naples, Florida 34109 (US); GUALDONI, John P., Naples, Florida 34109-7118 (US)
(74) Representative: Rankin, Douglas
(86) International application number: PCT/US2016/047234
(87) International publication number: WO 2017/040038

(56) References cited:
- WO-A1-99/37219
- WO-A1-2006/135935
- WO-A2-2013/052807
- US-A1- 2007 168 036
- US-A1- 2011 152 868
- US-A1- 2011 166 579
- US-A1- 2011 282 387
- US-A1- 2014 277 185
- US-A1- 2015 196 342

## Description

### BACKGROUND

This disclosure relates to a joint distraction system according to claim 1. Also described herein are surgical methods and devices for internal joint distraction.

In particular, the device and method described herein utilize less force and cause less collateral damage than existing joint distraction systems. Document US 2007/168036 A1 ("Ainsworth") discloses spinal motion preservation assemblies adapted for use in a spinal motion segment, while document WO 99/37219 A1 ("Orthodyne Inc") discloses a tissue anchoring system.

### SUMMARY

Disclosed herein are medical devices and examples of methods for internally distracting joints by using minimally invasive distraction. Minimally invasive devices are deployed within a portion of a bone proximal to a bone joint, e.g., in the greater trochanter, and which allow direct force application to the joint to be distracted. The disclosed joint distraction systems and devices are used in medical procedures for treating/correcting/ repairing damage/diseased articular joints, e.g., avascular necrosis of the hip, arthritis in younger people, femoral-cetabular impingements, etc.

In an illustrative example, a method of distracting a bone joint is disclosed and includes forming a passage or tunnel in one bone of a bone joint; arranging an anchor assembly in the passage; and applying a force directly to the anchor assembly to separate and distract the one bone from another bone of the bone joint. Another exemplary method includes distracting the bone joint between the femoral head and the acetabulum or the glenohumeral joint.

In an illustrative embodiment, a joint distraction system includes an anchor assembly including a cannulated fastener and a force applicator inserted through the cannulated fastener. The force applicator is rotatable relative to the cannulated fastener to distract a first bone of a joint from a second bone of the joint.

In an illustrative example, a joint distraction system includes an anchor assembly including an anchor device and a suture carried by the anchor device, and a force applicator system connectable to the suture and movable to apply a distraction force to the suture to distract a first bone of a joint away from a second bone of the joint.

### BRIEF DESCRIPTION OF THE DRAWINGS

FIG. 1 is a perspective view of an anchor assembly.
FIG. 2 is a perspective view of the anchor assembly of FIG. 1, deployed in the greater trochanter.
FIG. 3 is a perspective view of a suture button anchor assembly, deployed in a transosseous tunnel.
FIG. 4 is a perspective view of a suture-anchor anchor assembly, deployed in a transosseous socket.
FIG. 5 is a perspective view of an anchor assembly suture attached to a force application device of a patient table.
FIG. 6 is a side perspective view of the force applicator system for a patient/surgical table that allows the pulling/tensioning vector to be changed.
FIG. 7 is a perspective view of an adapter for attaching a force application system to a surgical/patient table rail.
FIG. 8 is a front perspective view of the surgical/patient table and force application system with a suture from an implant loaded onto the hook of the reel/spool device of the force application system.

### DETAILED DESCRIPTION

There is a need for an internal joint distracter and procedures which act to open and close the joint with lower force loads while avoiding peripheral nerve and tissue damage. Disclosed herein are medical devices and examples of methods for internally distracting joints by using minimally invasive distraction. Distraction devices as disclosed herein can be used in medical procedures for treating, correcting, and/or repairing damage/diseased articular joints, e.g., avascular necrosis of the hip, arthritis in younger people, femoral-acetabular impingements (a condition where the hip bones have an abnormal shape), or Chondrolysis (gradual degradation of hyaline cartilage in the hip joint), worn or diseased aspects of the bones forming the joint.

In a disclosed embodiment, a distraction device is installed within a bone of a bone joint and force is applied to or by the device to distract the joint bones and separate and space articular surfaces from a joint socket.

In another disclosed embodiment, an anchor assembly is secured temporarily within or against a bone of a bone joint and force is applied to the anchor assembly to distract the joint bones and separate and space articular surfaces from a joint socket.

In another disclosed embodiment, a tunnel is formed and can extend completely through a portion of one bone, and a distraction device is installed in the tunnel.

In another disclosed embodiment, twisting/rotating a component of a distraction device, e.g., a fastener, distracts the joint bones.

In another disclosed embodiment, applying a rotating force to a distraction device distracts joint bones.

In another disclosed example, applying a pulling force to a distraction device distracts joint bones.

In another disclosed embodiment for distracting a bone joint, a passage is formed in one bone of a bone joint, an anchor assembly is arranged within the passage, and a force is directly applied to a component of the anchor assembly to separate and distract the bones of the joint.

In another disclosed example for distracting a bone j oint, a tunnel passage is formed in one bone of a bone joint, an anchor assembly is arranged within the tunnel passage, and a force is directly applied to a suture component of the anchor assembly to separate and distract the bones of the bone joint.

In another disclosed example for distracting a bone joint, a bone socket is formed in one bone of a bone joint, an anchor assembly is arranged in the socket, and a force is directly applied to a suture component of the anchor assembly to separate and distract the bones of the bone joint

In another disclosed embodiment, an anchor assembly for joint distraction is installed in the greater trochanter.

In another disclosed embodiment, an anchor assembly for joint distraction is installed in the distal epiphysis of a bone.

In another disclosed embodiment, twisting/rotating a threaded shaft within a threaded cannulation distracts a joint.

In another disclosed embodiment, a cannulated fastener is inserted and lodged in the greater trochanter, and a blunt, threaded screw or peg is inserted and twisted flush to the anterior inferior iliac spine on the pelvis. The threaded screw or peg is then twisted clockwise to achieve hip joint distraction.

In another disclosed example, a suture-anchor construct is deployed in the greater trochanter, and the suture is externally tensioned to distract the joint. The sutures can be attached to a tensioner on a treatment surgical/patient/table or bed which directly applies tension to the suture.

In another disclosed example, a suture-anchor construct is deployed in the transosseous tunnel/socket, and the suture is externally tensioned to distract the joint. The sutures can be attached to a tensioner on a treatment surgical/patient/table or bed which directly applies tension to the suture.

In another disclosed example, the tensioner is a force application system that is capable of five degrees of motion, and permits positional adjustments along the x-, y- and z- axes, and angularly and radially.

In another disclosed example, a suture anchor is provided, e.g., the anchor is externally threaded and inserted into the lateral aspect of the greater trochanter. The sutures are then attached to a treatment table or bed with an external tensioning device which directly applies tension to the suture.

In another disclosed example, a distraction procedure involves inserting and passing an anchor/button construct which includes a suture/cable through a hole in joint bone and applying force to the suture/cable by a force application device, such as a spool or reel, to directly apply a distracting force to the bone.

In another disclosed example, a distraction procedure includes an anchor or button construct with heavy gauge suture or cable, which is passed through a prepared tunnel and affixed to the bone. The suture/cable can be percutaneously removed and attached to a force application system, such as a spool or reel statically mounted to a rail adapter of a surgical/patient table. A handle/crank or other suitable system can be used to rotate the spool or reel in a clockwise or counterclockwise direction to increase or decrease the amount of force applied to the suture/cable. Instead of a user controlled system, an automated system can be provided.

In another disclosed example of a distraction procedure, a tunnel is prepared in a joint bone, an anchor or button construct with heavy gauge suture or cable is passed through the prepared tunnel, the anchor/button is affixed to the bone, and suture or cable is percutaneously removed and attached to a force/tension application device (force applicator), such as a spool or reel statically mounted on a surgical table, such as a Clark rail adapter/clamp, associated with a patient table.

In another disclosed embodiment, a sheath is inserted into a joint through the greater trochanter via a drilled pilot hole. Then, a blunt tipped screw is inserted through that sheath until it reaches the anterior inferior iliac spine on the acetabulum. The screw is twisted in a clockwise direction, and the femoral head is distracted away from the acetabulum. The medical procedure is completed in the joint and the screw and sheath are removed

In another disclosed embodiment, a distraction kit includes at least one distraction device which includes a cannulated fastener (anchor/sheath), a set of different sized cannulated fasteners, a force applicator or set of force applicators receivable within corresponding cannulations, and drive tools to engage the fastener and/or force applicator.

In another disclosed example, a distraction kit includes at least one of a first distraction device, a second distraction device, and a third distraction device, wherein the first, second and third distraction devices are the same or different, and one distraction device includes a cannulated anchor and force applicator, and the second and third distraction devices include suture anchor constructs.

These and other embodiments of this disclosure will become apparent from the following detailed description when read in conjunction with the accompanying drawings and illustrated exemplary embodiments.

In the following detailed description, reference is made to various embodiments. It is to be understood that other embodiments may be employed, and that structural and logical changes may be made without departing from the scope of the disclosure.

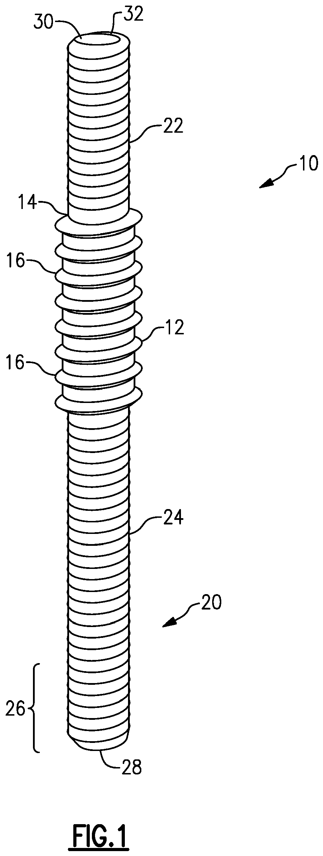

FIG. 1 shows a first distraction device which includes anchor assembly 10 with a cannulated fastener 12 (anchor or sheath) component/element having internal threads 14 and external threads 16. The anchor assembly 10 uses less force and causes less collateral damage than existing joint distraction systems. A threaded element 20 (force applicator or jack), e.g., a rod/shaft/screw/peg, is another component/element of the anchor assembly 10, and includes a shaft 22, with external threads 24, and a distal tip 26 that has a blunt and non-threaded end 28. The threaded element 20 includes a proximal end 30 that can be headless or include a head each of which is configured (32) to be engaged by a drive tool having a similarly configured tip, hex, star, square, etc. (not shown). Although the force applicator is illustrated to be a single/unitary externally threaded member, the force applicator can comprise a series of the same of different length sections that have complimentary distal and proximal ends to engage one another and provide an applicator whose length can be patient adjusted.

The external threads 16 of the cannulated fastener 12 are configured to permit rotational insertion, lodging, and removal of the cannulated fastener 12 within the greater trochanter or bone adjacent the bone joint to be distracted. The internal threads 14 correspond to and cooperate with the external threads 24 of the threaded element 20 so that rotation of the threaded element 20 advances the threaded element 20 through the cannulated fastener 12. One or more anchor assemblies 10 can be provided in a kit which includes different sized cannulated fasteners, the same of different sized threaded elements 20, and drive tools to engage and rotate the cannulated fasteners 12 and/or the threaded element 20.

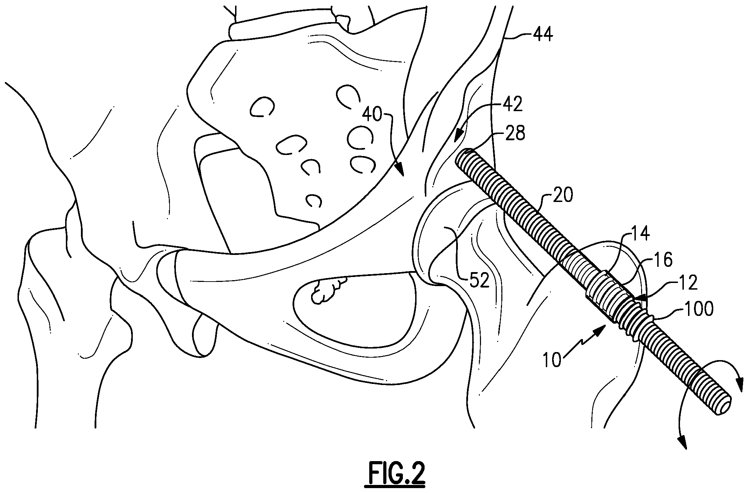

FIG. 2 illustrates distraction with the cannulated fastener 12 that is inserted into a transosseous tunnel 100 in and through the greater trochanter. The tunnel 100 is prepared with a drill guide assembly, such as that used in various orthopedic procedures to efficiently and accurately position other surgical instrumentation relative to bone. A drill guide assembly that is used to prepare a hip joint for reconstructing the *ligamentum teres,* or other orthopedic procedures, including but not limited to, bone resurfacing procedures, joint replacement procedures, or other ligament reconstruction procedures, can be used. Suitable drill guides that confer the surgeon the ability to target bones from outside the joint, involving an alignment bar, such as that disclosed in U.S. Patent Publication No. 2014/0276841 or an articulating drill guide, such as that disclosed in U.S. Patent Publication No. 2014/0114322, may be used.

Once the transosseous tunnel 100 has been formed, rigid cannulated fastener 12 having internal threads 14 and external threads 16 is positioned, e.g., screwed into the tunnel 100. With the anchor assembly 10 illustrated in FIGS. 1 and 2, the cannulated fastener 12 is rotated until it is fixedly positioned within the tunnel 100 to a depth sufficient to permit the cannulated fastener 12 to function as a leverage point. Thereafter the blunt ended threaded element 20 is threaded into the cannulated fastener 12 until end 28 engages the anterior inferior iliac spine 42 on the acetabulum 44. Further clockwise rotation/twisting of the threaded element 20 separates the femoral head 52 from the anterior inferior iliac spine 42 on the acetabulum 44 to distract the joint 40. Once distracted, a medical practitioner may perform a desired medical procedure. Upon completion of the medical procedure, the threaded element 20 is twisted/rotated counter-clockwise to return the distracted femoral head to pre-surgical positions.

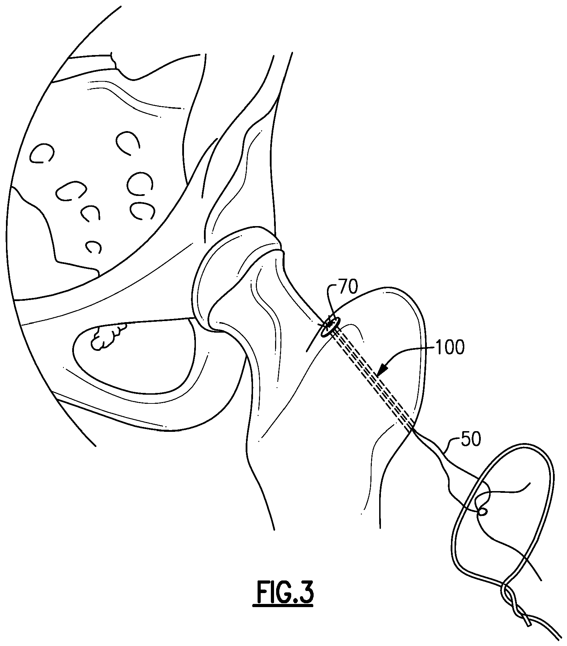

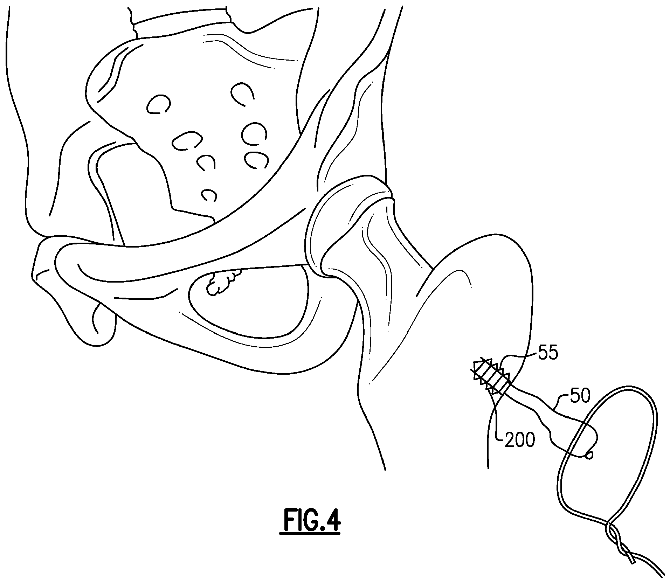

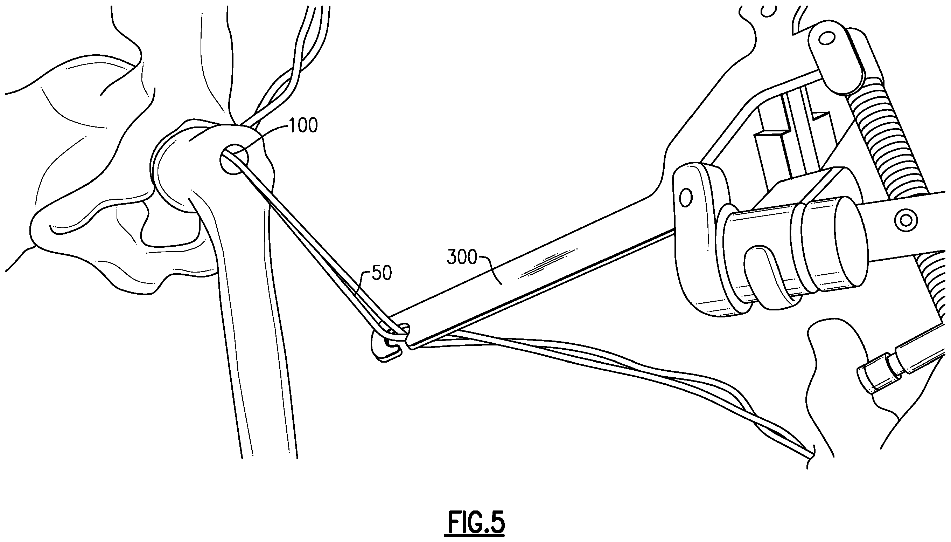

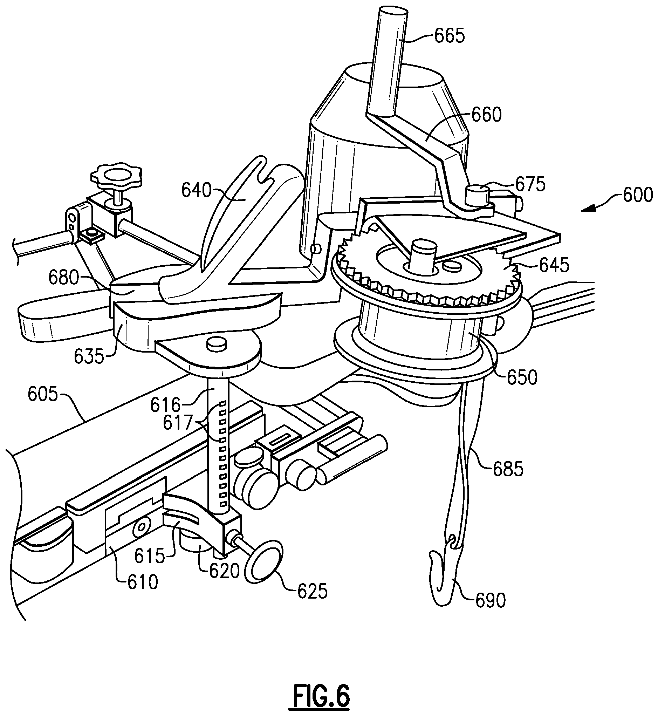

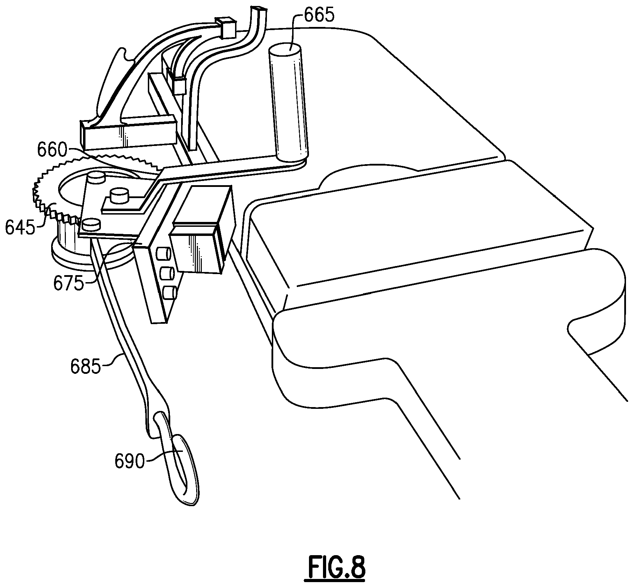

In FIGS. 3 and 4 joint distraction utilizing less force and causing less collateral damage than existing joint distraction systems is accomplished by using anchor assemblies 110 that include a suture 50, e.g., the sutures can be affixed to the bone in any suitable fashion, for example with a button 70 having a first minor dimension sufficient to pass through the tunnel and a second major dimension sufficient to span the tunnel diameter, e.g., BicepsButton™ (FIG.3), to engage a distal side of the bone so that the joint can be distracted when the suture 50 is pulled. A small transosseous tunnel 100 to pass the suture 50 and the button 70 combination (FIGS. 3 and 5), or socket 200 for a suture anchor 55 (FIG. 4) (e.g., a Corkscrew® anchor) is prepared. Suitable drill guides that confer the surgeon the ability to target bones from outside the joint, involving an alignment bar, such as that disclosed in U.S. Patent Publication No. 2014/0276841 or an articulating drill guide, such as that disclosed in U.S. Patent Publication No. 2014/0114322, may be used. The sutures 50 exit percutaneously and are attached to a distraction device, as shown in FIGS. 5-7. The distraction device (e.g., cable/suture end of the BicepsButton™ or Corkscrew® anchor) is attached to a bed rail component 300 of a patient table (FIG. 5), or hook 690 (FIGS. 6 and 8) and is pulled by a spool or reel mechanism, such as a Clark rail reel mechanism that can be adjusted up/down and/or side by side to achieve ideal distraction angle.

FIGS. 6-8 illustrate a force applicator system 600 associated with a surgical/patient table 605. In FIG. 6, the force applicator system 600 is secured to the patient table 605 by rail clamp 615 which can slide onto and along a Clark rail 610. The force applicator system 600 also includes a reel/spool housing or reel 645, a housing mounting frame 675, and a platform 635 upon which the frame 675 is located. The frame 675 supports the reel 645 for rotation, and the platform 635 supports the frame 675 for both reciprocal and angular repositioning to adjust the force vector for the reel/spool wire/cable. The frame 675 includes a handle 640 which reciprocally slides within a channel on the platform 635 of the frame 675.

The rail clamp 615 includes a vertical hole through which post 616 extends. Post 616 is provided with a plurality of equally-spaced holes or openings 617 that allow more manual adjusting of the height of the device (relative to the Clark rail 610, for example) by a pulling knob 625 that include a shaft (not shown) that can be moved into and out of the openings 617. The pulling knob 625, e.g., a height adjustment knob, can be of any geometry, including round, oval, or scalloped, to allow the vertical position of the surgical leg positioning device to be adjusted as needed.

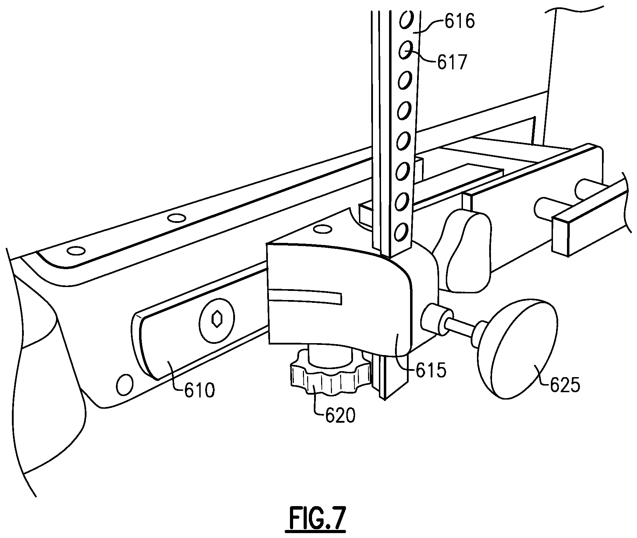

FIG. 7 shows the close-up view of the rail clamp 615, which also includes knob 620 which allows the rail clamp 615 and post 616 to move axially along the Clark rail 610. This adjustment can be effected prior to tightening the rail clamp 615 by the manual tightening of knob 620 which locks the post and supported components in a desired position. The knob 620 may have a scalloped configuration, for ease of manipulation. The knob 620 allows the rail clamp 615 to slide along the Clark rail 610 until in the desired position and then tightened to secure the rail clamp 615 in position for securing and unsecuring the rail clamp 615 from the Clark rail 610 for movement along the surgical/patient table.

Referring again to FIG. 6, the platform 635 is located at the top of the post 616. The vertical position of the platform 635 can be adjusted by pulling on the pulling knob 625. The pulling knob 625 includes a cylindrical shaft (not shown) that is positionable within any one of the plurality of holes 617 in the post 616 by pulling/withdrawing the knob and moving the platform in the vertical direction.

The platform 635 includes upper and lower sections that can rotate relative to one another, and includes a base plate (lower plate or section) that is connected to a rotational positioning plate (upper plate or section) by a swivel lock (not shown) which when loosened by manual/automatic operation, allows the rotational positioning base to articulate with respect to the base plate and to pivot or rotate the frame 675 and the reel 645. Post 616, described above, are rigidly connected to base/lower plate/section which positions and supports in cantilever fashion rotational positioning upper/base. The rotational base plate, e.g., upper plate, includes open channel 680 for locating therein an arm of the frame 675. A locking handle 640 extends from a proximal end of the frame arm and permits repositioning movement along the channel. Platform 635 provides for both reciprocating and angular repositioning of the reel/spool housing.

Reel 645 of a cable (or wire) 650 is rotatably mounted on the platform 635, and can be rotated or turned via handle 665 which extends from the crank 660. Any other suitable device, instead of the mechanical-user controlled reel rotation can be used, such as an automated system. In FIG. 8, the reel 645 is mounted for rotation within a frame 675. Cable 650 on the reel 645 is attached to a proximal end of a tether/strap 685. A hook 690 is arranged at the distal end of the tether/strap 685.

The suture 50 from anyone of the above examples can be connected to the hook 690 and the handle 665 of the crank 660 rotated to increase tension on the sutures 50 thereby pulling the femoral head out of the hip joint. Rotating the reel 645 in the opposite direction releases tension on the sutures 50 so that the femoral head can return to its natural positon within the hip joint.

Although the present disclosure has been described in connection with preferred embodiments, many modifications and variations will become apparent to those skilled in the art. It should be understood that the above disclosure and embodiments therein are exemplary and are not to be considered as limiting.

## Claims

1. A joint distraction system, comprising:
an anchor assembly (10) including a cannulated fastener (12) and a force applicator (20) inserted through the cannulated fastener (12), and the force applicator (20) is rotatable relative to the cannulated fastener (12) to distract a first bone of a joint from a second bone of the joint,
wherein the cannulated fastener (12) is insertable into a first bone, wherein the cannulated fastener (12) has an internal thread (14) adapted to engage an external thread (16) of the force applicator (20),
**characterised in that**
the force applicator (20) includes a blunt, non-threaded distal end (28) that extends from the cannulated fastener (12) and is configured to abut against a second bone, such that when the cannulated fastener (12) is fixedly positioned within the first bone, the force applicator (20) can be rotated relative to the cannulated fastener (12) until the blunt, non-threaded distal end (28) of the force applicator (20) abuts against the second bone, and such that further rotation of the force applicator (20) relative to the cannulated fastener (12) distracts the first bone from the second bone.

2. The joint distraction system as recited in claim 1, wherein the cannulated fastener (12) is a cannulated anchor or a cannulated sleeve.

3. The joint distraction system as recited in claim 1, wherein the force applicator (20) is a rod, shaft, screw, or peg.

## Patentansprüche

1. Gelenkdistraktionssystem, umfassend:
eine Ankeranordnung (10), die ein kanüliertes Befestigungselement (12) und einen durch das kanülierte Befestigungselement (12) eingeführten Kraftapplikator (20) umfasst, wobei der Kraftapplikator (20) relativ zu dem kanülierten Befestigungselement (12) drehbar ist, um einen ersten Knochen eines Gelenks von einem zweiten Knochen des Gelenks zu distrahieren,
wobei das kanülierte Befestigungselement (12) in einen ersten Knochen einführbar ist, wobei das kanülierte Befestigungselement (12) ein Innengewinde (14) aufweist, das zum Eingreifen in ein Außengewinde (16) des Kraftapplikators (20) angepasst ist, **dadurch gekennzeichnet, dass**
der Kraftapplikator (20) ein stumpfes gewindeloses distales Ende (28) umfasst, das sich von der kanülierten Befestigungsvorrichtung (12) erstreckt und derart konfiguriert ist, dass es an einem zweiten Knochen anliegt, sodass, wenn die kanülierte Befestigungsvorrichtung (12) fest in dem ersten Knochen positioniert ist, der Kraftapplikator (20) relativ zu dem kanülierten Befestigungselement (12) gedreht werden kann, bis das stumpfe gewindelose distale Ende (28) des Kraftapplikators (20) an dem zweiten Knochen anliegt, und dass eine weitere Drehung des Kraftapplikators (20) relativ zu dem kanülierten Befestigungselement (12) den ersten Knochen von dem zweiten Knochen distrahiert.

2. Gelenkdistraktionssystem nach Anspruch 1, wobei das kanülierte Befestigungselement (12) ein kanülierter Anker oder eine kanülierte Hülse ist.

3. Gelenkdistraktionssystem nach Anspruch 1, wobei der Kraftapplikator (20) ein Stab, ein Schaft, eine Schraube oder ein Zapfen ist.

## Revendications

1. Système d'écartement d'articulation, comprenant :
un ensemble d'ancrage (10) comprenant une attache canulée (12) et un applicateur de force (20) inséré à travers l'attache canulée (12), et l'applicateur de force (20) peut tourner par rapport à l'attache canulée (12) pour écarter un premier os d'une articulation d'un deuxième os de l'articulation,
dans lequel l'attache canulée (12) est insérable dans un premier os, dans lequel l'attache canulée (12) a un filetage interne (14) adapté pour s'engager avec un filetage externe (16) de l'applicateur de force (20), **caractérisé en ce que**
l'applicateur de force (20) comprend une extrémité distale arrondie et non filetée (28) qui s'étend à partir de l'attache canulée (12) et est configurée pour venir en butée contre un deuxième os, de sorte que lorsque l'attache canulée (12) est positionnée de manière fixe à l'intérieur du premier os, l'applicateur de force (20) peut être tourné par rapport à l'attache canulée (12) jusqu'à ce que l'extrémité distale arrondie et non filetée (28) de l'applicateur de force (20) bute contre le deuxième os, et de telle sorte qu'une rotation supplémentaire de l'applicateur de force (20) par rapport à l'attache canulée (12) écarte le premier os du deuxième os.

2. Système de distraction articulaire selon la revendication 1, dans lequel la fixation canulée (12) est un ancrage canulé ou un manchon canulé.

3. Système d'écartement d'articulation selon la revendication 1, dans lequel l'applicateur de force (20) est une tige, un arbre, une vis ou une cheville.
